(19) Europäisches Patentamt
European Patent Office
Office européen des brevets

(11) Veröffentlichungsnummer: **0 014 734**
**A1**

# (12) EUROPÄISCHE PATENTANMELDUNG

(21) Anmeldenummer: **79103253.5**

(22) Anmeldetag: **03.09.79**

(51) Int. Cl.³: **C 07 D 249/12**
A 01 N 43/64

(30) Priorität: **06.09.78 CH 9374/78**
**11.04.79 CH 3481/79**
**09.08.79 CH 7310/79**

(43) Veröffentlichungstag der Anmeldung:
**03.09.80 Patentblatt 80/18**

(84) Benannte Vertragsstaaten:
**AT BE CH DE FR GB IT NL**

(71) Anmelder: **CIBA-GEIGY AG**
**Patentabteilung Postfach**
**CH-4002 Basel(CH)**

(72) Erfinder: **Kristiansen, Odd, Dr.**
**Delligrabenstrasse 7**
**CH-4313 Möhlin(CH)**

(72) Erfinder: **Drabek,Jozef,Dr.**
**Benkenstrasse 12**
**CH-4104 Oberwil(CH)**

(74) Vertreter: **Zumstein sen., Fritz, Dr. et al,**
**Bräuhausstrasse 4**
**D-8000 München 2(DE)**

## (54) 1-N,N-Dimethylcarbamoyl-3(5)-alkyl-5(3)-alkylthioalkylthio-1,2,4-triazole, Verfahren zu ihrer Herstellung, diese Verbindungen enthaltende Mittel und ihre Verwendung zur Bekämpfung von Schädlingen sowie ihre Ausgangsprodukte und deren Herstellung.

(57) Verbindungen der Formel IA und IB

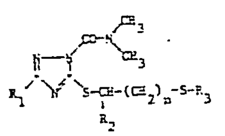

(IA)

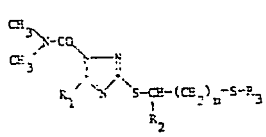

(IB)

worin $R_1$ i-Propyl, s-Butyl, t-Butyl oder gegebenenfalls durch Methyl substituiertes Cyclopropyl, $R_2$ Wasserstoff oder Methyl, $R_3$ $C_1$-$C_4$-alkyl und n die Zahl 0 oder 1 bedeuten mit pestizider vor allem insektizider Wirkung.

Zwischenprodukten für diese Verbindungen und Verfahren zu ihrer Herstellung.

EP 0 014 734 A1

Croydon Printing Company Ltd.

5-12017/1-3

1-N,N-Dimethylcarbamoyl-3(5)-alkyl-5(3)-alkylthioalkylthio-1,2,4-triazole, Verfahren zu ihrer Herstellung, Mittel welche diese Triazole enthalten und ihre Verwendung zur Bekämpfung von Schädlingen.

Die vorliegende Erfindung betrifft neue 1-N,N-Dimethylcarbamoyl-3(5)-alkyl-5(3)-alkylthioalkylthio-1,2,4-triazole, welche eine Wirkung gegen Schädlinge besitzen, und ein Verfahren zu ihrer Herstellung sowie Schädlingsbekämpfungsmittel, welche die neuen Verbindungen als aktive Komponente enthalten, und Verfahren zur Bekämpfung von Schädlingen unter Verwendung der genannten Triazole.

1-N,N-Dialkylcarbamoyl-3(5)-alkyl-5(3)-hydrocarbylthio-1,2,4-triazole mit pestizider vor allem insektizider Wirkung sind bekannt (siehe z.B. US Patent Nr. 3,308,131 und 4,066,774, sowie britisches Patent Nr. 1,510,636). Nach vorliegender Erfindung werden neuartige Verbindungen dieses Typus bereitgestellt, die eine besonders gute Wirkung gegen Insekten besitzen und welche aufgrund ihrer vorteilhaften biologischen Eigenschaften für die praktische Anwendung besonders geeignet sind.

Die erfinundgsgemässen, neuen 1-N,N-Dimethylcarbamoyl-3(5)-alkyl-5(3)-alkylthioalkylthio-1,2,4-triazole entsprechen der Formel IA bzw. IB

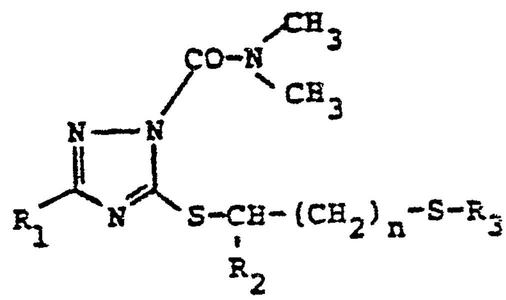

(IA)

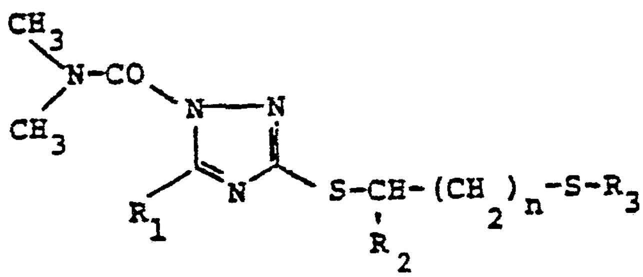

(IB)

worin $R_1$ eine i-Propyl-, s-Butyl-, t-Butyl- oder gegebenenfalls durch Methyl substituierte Cyclopropylgruppe, $R_2$ ein Wasserstoffatom oder eine Methylgruppe, $R_3$ eine $C_1$-$C_4$-Alkylgruppe und n die Zahl 0 oder 1 bedeuten.

Die für $R_3$ in Frage kommenden Alkylgruppen sind die Methyl-, Aethyl-, n-Propyl- und i-Propylgruppe sowie die n-, i-, s- und t-Butylgruppe.

In den Verbindungen der Formel IA und IB werden die folgenden Substituententypen sowie Kombinationen derselben untereinander bevorzugt:

1) Bei $R_1$ : Propyl, t-Butyl, Cyclopropyl, 1-Methyl-cyclopropyl und 2-Methyl-cyclopropyl insbesondere t-Butyl;

2) Bei $R_2$: Methyl und Aethyl, insbesondere Methyl.

Die erfindungsgemässen Verbindungen können in Form von Isomeren der oben dargestellten Formel IA und IB vorliegen. Nach dem hierin beschriebenen Herstellungsverfahren werden Gemische dieser zwei Isomeren erhalten, wobei in gewissen Fällen (z.B. bei Verbindungen,

worin $R_1$ t-Butyl oder 1- bzw. 2-Methyl-cyclopropyl bedeutet und insbesondere diejenigen, worin $R_2$ Wasserstoff darstellt) der überwiegende Anteil des Gemisches bzw. annähernd das ganze Produkt aus dem 3-Alkyl-5-alkylthioalkylthio-Isomeren der Formel IA besteht. Solche Isomerengemische können nach bekannten Methoden (z.B. durch chromatographische Trennung) in die einzelnen isomeren Formen aufgetrennt werden. Die beiden Isomeren der Formel IA und IB werden jedoch zweckmässig stets in Form ihrer nach dem beschriebenen Herstellungsverfahren hergestellten, unaufgetrennten Gemischen verwendet. Unter dem Begriff der gegenwärtigen Erfindung sind demzufolge sowohl die einzelnen Isomeren der Formel IA und IB als auch Gemische derselben zu verstehen.

Die erfindungsemässen Verbindungen der Formel IA und IB zeichnen sich durch eine ausgezeichnete insektizide Wirkung aus. Insbesondere weisen die erwähnten Verbindungen sowohl eine sehr gute systemische als auch Kontakt-Wirkung gegen saugende Insekten z.B. der Ordnung Homoptera und von allem der Familie Aphididae (wie z.B. Aphis fabae, Aphis craccivora und Myzus persicae) auf.

Ebenfalls besitzen die erfindungsgemässen Substanzen eine besonders starke Aktivität gegen Insekten der Ordnung Coleoptera So wurde es beispielsweise festgestellt, dass die Verbindungen der Formel IA und IB eine verglichen mit dem bekannten insektizid wirksamen 1-N,N-Dimethylaminocarbamoyl-3-t.-butyl-5-methylthio-1,2,4-triazol (vgl. U.S. Patent No. 1,066,774) deutlich überlegene Wirkung gegen Insekten-Schädlinge der Spezies Leptinotarsa decemlineata und Anthonomus grandis aufweisen. In diesem Zusammenhang ist auch die vorteilhafte Wirkung der erfindungsgemässen Verbindungen gegen Bodeninsekten vor allem gegen Bodeninsekten der letzteren genannten Ordnung (Coleoptera) besonders zu erwähnen.

Darüber hinaus zeigen die erfindungsgemässen Verbindungen auch eine Wirkung gegen pflanzenschädigende Akariden (Milben) z.B. der Familien Tetranychidae und Tyroglyphidae.

Die Verbindungen der Formel IA und IB sind demzufolge erfindungsgemäss zur Bekämpfung von Pflanzenschädigenden Insekten in Kulturen von Nutz- und Zierpflanzen, insbesondere in Baumwolle-, Obst- und Gemüsekulturen, besonders geeignet.

Die Verbindungen der Formel IA und IB werden analog bekannten Verfahren hergestellt, in dem man z.B. eine Verbindung der Formel II

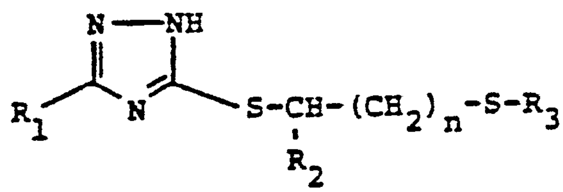

(II)

worin $R_1$, $R_2$, $R_3$ und n die für Formel IA und IB bereits angegebenen Bedeutungen haben in Gegenwart einer Base mit einem N,N-Dimethylcarbamoylhalid, insbesondere N,N-Dimethylcarbamoylchlorid reagieren lässt.

Das Verfahren wird zweckmässig bei einer Temperatur zwischen 30° und 150°C, meist zwischen 40° und 80°C, bei normalem oder leicht erhöhtem Druck und vorzugsweise in Gegenwart eines gegenüber den Reaktionsteilnehmern inerten Lösungs- oder Verdünnungsmittels durchgeführt.

Als Lösungs- oder Verdünnungsmittel eignen sich z.B. Ketone wie Aceton, Methyläthylketon und Cyclohexanon sowie Acetonitril.

Als für dieses Verfahren geeignete Basen kommen insbesondere tertiäre Amine, wie Trialkylamine, Pyridine und Dialkylaniline; ferner Hydroxide, Oxide, Carbonate und Bicarbonate von Alkali- und Erdalkalimetallen; sowie Alkalimetallalkoholate wie z.B. Kalium-tert.butylat und Natrium-methylat in Betracht.

Die Ausgangsstoffe der Formel II sind neu und gehören ebenfalls zum Erfindungsgegenstand. Sie können analog bekannten Methoden aus bereits vorhandenen Vorläufern dadurch erhalten werden, indem man z.B. eine Verbindung der Formel III

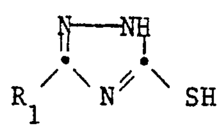

(III)

in Gegenwart einer Base (wie z.B. $NaOC_2H_5$) mit einer Verbindung der Formel IV

$$Hal - CH(R_2) - (CH_2)_n - S - R_3 \qquad (IV)$$

umsetzt, wobei in den Formeln III und IV, $R_1$ bis $R_3$ und n die unter Formel I bereits angegebenen Bedeutungen haben und Hal für ein Halogenatom steht.

Das Verfahren zur Herstellung des Ausgangstoffes wird zweckmässig bei einer Temperatur zwischen 60 und 100°C und vorzugsweise in Gegenwart eines gegenüber den Reaktionsteilnehmern inerten Lösungs- oder Verdünnungsmittels wie z.B. Aethanol durchgeführt.

Die Verbindungen der Formel IA und IB und deren Gemische werden erfindungsgemäss als solche verwendet oder bilden einen Bestandteil von Mitteln, welche noch geeignete Trägerstoffe oder Zuschlagstoffe oder Gemische solcher Stoffe enthalten.

Geeignete Träger und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die insektizide bzw. akarizide Wirkung der erfindungsgemässen Mittel lässt sich durch Zusatz anderer Akarizide und/oder Insektizide wesentlich verbreitern.

Als Zusätze eignen sich z.B.: org. Phosphorverbindungen; Nitrophenole und deren Derivate; Formamidine; Harnstoffe; pyrethrinartige Verbindungen; Karbamate und chlorierte Kohlenwasserstoffe.

Die erfindungsgemässen Mittel können z.B. als Stäubemittel, Dispersionen, Lösungen und Aufschlämmungen sowie als in Wasser dispergierbare Spritzpulver, Pasten, Emulsionen und Emulsionskonzentrate vorliegen und angewendet werden. Vorzugsweise aber werden die genannten Mittel als Granulate (z.B. Umhüllungsgranulate, Imprägnierungsgranulate und Homogengranulate), welche zum Streuen auf die Bodenoberfläche besonders geeignet sind, formuliert.

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneten Applikationsgeräte auch höhere Konzentrationen eingesetzt werden können.

Die erfindungsgemässen Verbindungen (Wirkstoffe) können beispielsweise wie folgt formuliert werden:

Granulat: Zur Herstellung eines 5%igen Granulates werden die folgenden Stoffe verwendet:

5 Teile Wirkstoff,
0,25 Teile Epichlorhydrin,
0,25 Teile Cetylpolyglykoläther
3,50 Teile Polyäthylenglykol,
91 Teile Kaolin (Korngrösse 0,3 - 0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Emulsionskonzentrat I

20 Gew.-Teile des obengenannten Wirkstoffes werden in
70 Gew.-Teilen Xylol gelöst und mit
10 Gew.-Teilen eines Emulgiermittels, bestehend aus einem Gemisch eines Arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

Emulsionskonzentrat II

5 bis max. 30 Gew.-Teile Wirkstoff werden unter Rühren bei Zimmertemperatur in
30 Gew.-Teilen Dibutylphthalat
10 Gew.-Teilen Solvent 200 (niederviskoses hocharomat. Erdöldestillat)
15 bis 35 Gew.- Teilen Dutrex 238 CF (viskoses hocharomat, Erdöldestillat) gelöst und mit
10 Gew.-Teilen eines Emulgatorgemisches, bestehend aus Ricinusölpolyglykoläther und dem Cacliumsalz der Dodecyl-

benzolsulfonsäure, versetzt. Das so erhaltene Emulsionskonzentrat gibt in Wasser milchige Emulsionen.

Spritzpulver

5 bis 30 Gew.-Teile des Wirkstoffes werden in einer Mischapparatur mit

| | |
|---|---|
| 5 Gew.-Teilen | eines aufsaugenden Trägermaterials (Kieselsäure K 320 oder Wessalon S) und |
| 55 bis 80 Gew.-Teilen | eines Trägermaterials (Bolus alba oder Kaolin B 24) und einem Dispergiermittelgemisch, bestehend aus |
| 5 Gew.-Teilen | eines Na-lauryl-sulfonates und |
| 5 Gew.-Teilen | eines Alkyl-aryl-polyglykoläthers, intensiv vermischt. Diese Mischung wird auf einer Stift- oder Lufstrahlmühle bis auf 5-15 µm gemahlen. Das so erhaltene Spritzpulver gibt in Wasser eine gute Suspension. |

Stäubemittel

| | |
|---|---|
| 2 Gew.-Teile | feingemahlener Wirkstoff werden mit |
| 1 Gew.-Teil | einer gefällten Kieselsäure und |
| 97 Gew.-Teilen | Talk intensiv vermischt. |

Die nachfolgenden Beispiele dienen zur näheren Erläuterung der Erfindung.

Beispiel 1 : Herstellung von 1-N,N-Dimethylcarbamoyl-3-t.-butyl-5-(2-methylthio-äthylthio)-1,2,4-triazol

a) Herstellung des Ausgangsstoffes:

Zu einer Lösung von 15,7 g 3-t.-Butyl-5-mercapto-1,2,4-triazol in äthanolischem Natriumäthylat (2,3 g Natrium in 300 ml Aethanol) wurden 15 g 2-Chloräthyl-methylsulfid zugetropft. Das Reaktionsgemisch wurde 2 Stunden bei 80°C gerührt und nach dem Abkühlen abfiltriert. Anschliessend wurde das Lösungsmittel unter Vakuum abgetrieben, und der Rückstand in 300 ml Chloroform aufgenommen und mit 100 ml Wasser ausgeschüttelt. Nach dem Abdestillieren des Lösungsmittels wurde das Rohprodukt mit kaltem Petroläther gewaschen. Auf diese Weise erhielt man das 3-t.-Butyl-5-(2-methylthio-äthylthio)-1,2,4-triazol der Formel

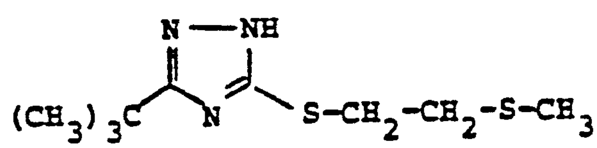

mit einem Smp. von 114-116°C.

Die nachfolgenden Verbindungen der vorstehend dargestellten Formel II sind auf analoge Weise erhältlich:

| $R_1$ | $R_2$ | n | $R_3$ | Physikalische Daten |
|---|---|---|---|---|
| i-$C_3H_7$ | H | 0 | $CH_3$ | Smp.: 122-124°C |
| i-$C_3H_7$ | H | 1 | $CH_3$ | Smp.: 95-97°C |
| i-$C_3H_7$ | $CH_3$ | 0 | $CH_3$ | |
| i-$C_3H_7$ | $CH_3$ | 1 | $CH_3$ | |

| $R_1$ | $R_2$ | n | $R_3$ | Physikalische Daten |
|---|---|---|---|---|
| $i\text{-}C_3H_7$ | H | 1 | $C_2H_5$ | |
| $i\text{-}C_3H_7$ | H | 0 | $i\text{-}C_3H_7$ | Smp.: 57-60°C |
| $i\text{-}C_3H_7$ | H | 1 | $i\text{-}C_3H_7$ | Smp.: 86-90°C |
| $i\text{-}C_3H_7$ | H | 0 | $s\text{-}C_4H_9$ | $n_D^{20}$ = 1,5386 |
| $t\text{-}C_4H_9$ | H | 0 | $CH_3$ | Smp.: 122-124°C |
| $t\text{-}C_4H_9$ | $CH_3$ | 0 | $CH_3$ | |
| $t\text{-}C_4H_9$ | $CH_3$ | 1 | $CH_3$ | Smp.: 108-112°C |
| $t\text{-}C_4H_9$ | H | 1 | $C_2H_5$ | Smp.: 98-102°C |
| $t\text{-}C_4H_9$ | $CH_3$ | 0 | $C_2H_5$ | Smp.: 139-142°C |
| $t\text{-}C_4H_9$ | $CH_3$ | 1 | $C_2H_5$ | Smp.: 88-92° |
| $t\text{-}C_4H_9$ | H | 0 | $i\text{-}C_3H_7$ | |
| $t\text{-}C_4H_9$ | H | 1 | $i\text{-}C_3H_7$ | Smp.: 118-121° |
| $t\text{-}C_4H_9$ | H | 1 | $n\text{-}C_4H_9$ | |
| $t\text{-}C_4H_9$ | H | 1 | $t\text{-}C_4H_9$ | |
| $s\text{-}C_4H_9$ | H | 0 | $CH_3$ | |
| $s\text{-}C_4H_9$ | H | 1 | $CH_3$ | Smp.: 57-58° |
| 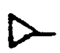 | H | 0 | $CH_3$ | Smp.: 80-85° |
| 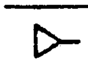 | H | 1 | $CH_3$ | Smp.: 98-100° |
| 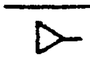 | $CH_3$ | 1 | $CH_3$ | |
| 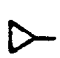 | H | 1 | $C_2H_5$ | |
| 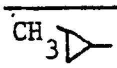 | H | 0 | $CH_3$ | Smp.: 184-186° |
| 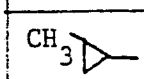 | H | 1 | $CH_3$ | Smp.: 168-170° |
| 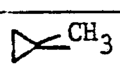 | H | 0 | $CH_3$ | Smp.: 88-94° |
| 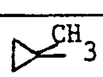 | H | 1 | $CH_3$ | Smp.: 88-91° |

b) <u>Herstellung des Endproduktes</u>

Zu einer Suspension von 11,6 g 3-t.-Butyl-5-(2-methylthio-äthylthio)-1,2,4-triazol und 7 g wasserfreiem Kaliumcarbonat in Aceton (200 ml) wurden 5,9 g Dimethylcarbamoylchlorid zugetropft. Das Gemisch wurde 4 Stunden unter Rückfluss gekocht und nach dem Abkühlen, Abfiltrieren und Eindampfen das Rohprodukt anschliessend in 200 ml Chloroform gelöst und mit 100 ml Wasser ausgeschüttelt. Nach dem Abdestillieren des Lösungsmittels im Vakuum und Waschen des Rohproduktes mit Petroläther erhielt man das 1-N,N-Dimethyl-carbamoyl-3-t.-butyl-5-(2-methylthio-äthylthio)-1,2,4-triazol der Formel

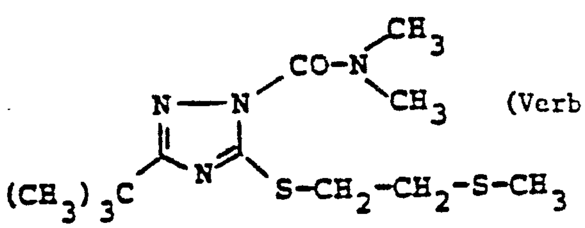

(Verbindung Nr. 1)

als weisse Kristalle mit einem Smp. von 42-43°C.

Die folgenden Verbindungen der vorstehend dargestellten Formel IA und IB können auf analoge Weise erhalten werden:

| Verb. Nr. | $R_1$ | $R_2$ | n | $R_3$ | Physikalische Daten |
|---|---|---|---|---|---|
| 2 | $i-C_3H_7$ | H | 0 | $CH_3$ | $n_D^{20}$: 1,5500 |
| 3 | $i-C_3H_7$ | H | 1 | $CH_3$ | $n_D^{20}$: 1,5467 |
| 4 | $i-C_3H_7$ | $CH_3$ | 0 | $CH_3$ | |
| 5 | $i-C_3H_7$ | $CH_3$ | 1 | $CH_3$ | |
| 6 | $i-C_3H_7$ | H | 1 | $C_2H_5$ | $n_D^{20}$: 1,5394 |
| 7 | $i-C_3H_7$ | H | 0 | $i-C_3H_7$ | $n_D^{20}$: 1,5372 |
| 8 | $i-C_3H_7$ | H | 1 | $i-C_3H_7$ | $n_D^{20}$: 1,5133 |
| 9 | $i-C_3H_7$ | H | 0 | $s-C_4H_9$ | $n_D^{20}$: 1,5347 |
| 10 | $t-C_4H_9$ | H | 0 | $CH_3$ | Smp.: 62-64°C |
| 11 | $t-C_4H_9$ | $CH_3$ | 0 | $CH_3$ | |
| 12 | $t-C_4H_9$ | $CH_3$ | 1 | $CH_3$ | $n_D^{20}$: 1,5353 |
| 13 | $t-C_4H_9$ | H | 1 | $C_2H_5$ | $n_D^{20}$: 1,5355 |
| 14 | $t-C_4H_9$ | $CH_3$ | 0 | $C_2H_5$ | $n_D^{20}$= 1,5284 |
| 15 | $t-C_4H_9$ | $CH_3$ | 1 | $C_2H_5$ | $n_D^{20}$: 1,5301 |
| 16 | $t-C_4H_9$ | H | 0 | $i-C_3H_7$ | |
| 17 | $t-C_4H_9$ | H | 1 | $i-C_3H_7$ | $n_D^{20}$: 1,5296 |
| 18 | $t-C_4H_9$ | H | 1 | $n-C_4H_9$ | |
| 19 | $t-C_4H_9$ | H | 1 | $t-C_4H_9$ | |
| 20 | $s-C_4H_9$ | H | 0 | $CH_3$ | $n_D^{20}$: 1,5430 |
| 21 | $s-C_4H_9$ | H | 1 | $CH_3$ | $n_D^{20}$: 1,5413 |
| 22 | 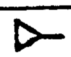 | H | 0 | $CH_3$ | $n_D^{20}$: 1,5761 |

| Verb. Nr. | $R_1$ | $R_2$ | n | $R_3$ | Physikalische Daten |
|---|---|---|---|---|---|
| 23 | 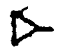 | H | 1 | $CH_3$ | Smp.: 39-41°C |
| 24 | 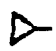 | $CH_3$ | 1 | $CH_3$ | $n_D^{20}$: 1,5620 |
| 25 | 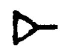 | H | 1 | $C_2H_5$ | $n_D^{20}$: 1,5615 |
| 26 | 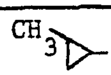 | H | 0 | $CH_3$ | $n_D^{20}$: 1,5654 |
| 27 | 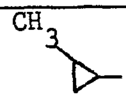 | H | 1 | $CH_3$ | $n_D^{20}$: 1,5604 |
| 28 | 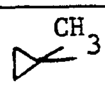 | H | 0 | $CH_3$ | $n_D^{20}$: 1,5660 |
| 29 | 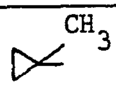 | H | 1 | $CH_3$ | $n_D^{20}$: 1,5599 |

Beispiel 2:

Insektizide Frass- und Kontaktwirkung: Anthonomus grandis

Baumwollpflanzen wurden mit einer wässrigen Emulsion enthaltend 0,05% der zu prüfenden Verbindung (erhalten aus einem 25%igen Spritzpulver) besprüht.

Nach dem Antrocknen des Belages wurden die Pflanzen mit Adulten der Spezies Anthonomus grandis besiedelt. Man verwendete zwei Pflanzen für jede Versuchsverbindung und eine Auswertung der erzielten Abtötung erfolgte 2,4,24 und 48 Stunden nach Versuchsbeginn.

Der Versuch wurde bei 24°C und 26% relativer Luftfeuchtigkeit durchgeführt.
Verbindungen gemäss Beispiel 1 zeigten im obigen Versuch eine gute Wirkung gegen Insekten der Spezies Anthonomus grandis.

Beispiel 3:

Insektizide Frass- und Kontaktwirkung: Leptinotarsa decemlineata.

Bei gleicher Arbeitsweise unter Verwendung von Kartoffelstauden anstelle von Baumwollpflanzen und Larven der Spezies Leptinotarsa decemlineata im L3-Stadium, wurde die im Beispiel 2 beschriebene Versuchsmethode wiederholt.

Verbindungen gemäss Beispiel 1 zeigten ebenfalls in diesem Versuch eine gute Wirkung gegen Larven der Spezies Leptinotarsa decemlineata.

Beispiel 4:

Insektizide Frass- und Kontaktwirkung: Aphis craccivora.

In Wasser angezogene Erbsen (Pisum sativum) wurden vor dem Versuchsbeginn je mit ca. 200 Individuen der Spezies Aphis craccivora besiedelt. Die so behandelten Pflanzen wurden 72 Stunden später mit einer Lösung enthaltend 200 oder 100 ppm der zu prüfenden Verbindung bis zur Tropfnässe besprüht.

Man verwendet pro Test-Verbindung und pro Konzentration zwei Pflanzen und eine Auswertung der erzielten Abtötungsrate erfolgte nach weiteren 24 Stunden.

Verbindungen gemäss Beispiel 1 zeigten im obigen Versuch eine sehr gute Wirkung gegen Insekten der Spezies Aphis craccivora.

Beispiel 5: Insektizide Wirkung: Pseudococcus citri

In Töpfen angezogene Pflanzen (Vicia faba), welche bis auf ein gut ausgebildetes Blattpaar zurückgeschnitten war, wurden 24 Stunden vor dem Versuchsbeginn mit ca. 200 Individuen der Spezies Pseudococcus citri besiedelt. Am nächsten Tag wurden die nun mit Läusen besetzten Unterseite der Blätter mit einer Versuchslösung enthaltend 500 ppm der zu prüfenden Verbindung bis zu Tropfnässe besprüht. Man verwendete pro Test-Substanz zwei Pflanzen und eine Auswertung der erzielten Abtötung erfolgte 4 bis 24 Stunden nach Versuchsbeginn.

Verbindungen gemäss Beispiel 1 wirkten im obigen Versuch gegen Pseudococcus citri.

Beispiel 6:

Insektizide Wirkung (systemisch): Aphis craccivora

Bewurzelte Bohnenpflanzen wurden in Töpfe, welche 600 ccm Erde enthalten, verpflanzt und anschliessend 50 ml einer Lösung der zu prüfenden Verbindung (erhalten aus einem 25%igen Spritzpulver) in einer Konzentration von 25 ppm. direkt auf die Erde aufgegossen.

Nach 24 Stunden wurden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgte 48 Stunden nach Versuchsbeginn. Pro Konzentrationsdosis Testsubstanz wurden zwei Pflanzen, je eine in einem separaten Topf, verwendet. Der Versuch wurde bei 25°C und 70% relativer Luftfeuchtigkeit durchgeführt.

Verbindungen gemäss Beispiel 1 zeigten im obigem Versuch eine gute systemische Wirkung gegen Insekten in Spezies Aphis craccivora.

**Beispiel 7:** Wirkung gegen pflanzenschädigende Akariden Tetranychus urticae (OP-sensible) und Tetranychus cinnabarinus (OP-tolerant)--

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt. (Die Toleranz bezieht sich auf die Verträglichkeit von Diazinon).

Die so behandelten infestierten Pflanzen wurden mit einer Versuchslösung enthaltend 400 oder 200 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25°C.

Verbindungen gemäss Beispiel 1 zeigen in diesem Versuch eine positive Wirkung gegen Individuen der Spezies Tetranychus urticae und Tetranychus cinnabarinus.

Patentansprüche

(für alle benannten Länder ausser Oesterreich)

1. Verbindungen der Formel IA bzw. IB

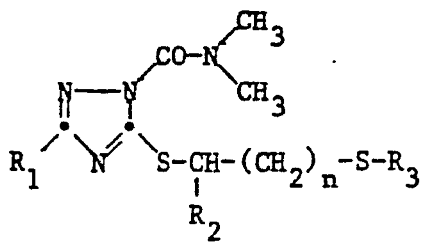

(IA)

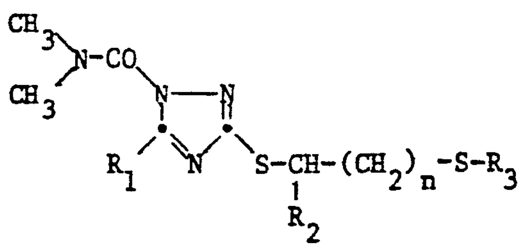

(IB)

worin $R_1$ eine i-Propyl-, s-Butyl-, t-Butyl- oder gegebenenfalls durch Methyl substituierte Cyclopropylgruppe, $R_2$ ein Wasserstoffatom oder eine Methylgruppe, $R_3$ eine $C_1$-$C_4$-Alkylgruppe und n die Zahl 0 oder 1 bedeuten und Gemische derselben.

2. Verbindungen nach Anspruch 1, worin $R_1$ eine i-Propyl-, s-Butyl-, t-Butyl- oder Cyclopropylgruppe bedeutet.

3. Verbindungen nach Anspruch 2, worin $R_1$ eine t-Butylgruppe bedeutet.

4. Verbindungen nach einem der Ansprüche 1 bis 3, worin $R_2$ ein Wasserstoffatom und $R_3$ eine Methyl- oder Aethylgruppe bedeuten.

5. Verbindungen nach einem der Ansprüche 1 bis 4, worin n die Zahl 0 bedeutet.

6. Verbindungen nach Anspruch 1, worin $R_1$ eine t-Butylgruppe, $R_2$ ein Wasserstoffatom, $R_3$ eine Methylgruppe und n die Zahl 0 bedeuten.

7. Verbindungen nach Anspruch 1, worin $R_1$ eine t-Butylgruppe, $R_2$ ein Wasserstoffatom, $R_3$ eine Methylgruppe und n die Zahl 1 bedeuten.

8. Verbindungen nach Anspruch 1, worin $R_1$ eine 2-Methyl-cyclopropylgruppe, $R_2$ ein Wasserstoffatom, $R_3$ eine Methylgruppe und n die Zahl 0 bedeuten.

9. Verbindungen nach Anspruch 1, worin $R_1$ eine 1-Methyl-cyclopropylgruppe, $R_2$ ein Wasserstoffatom, $R_3$ eine Methylgruppe und n die Zahl 1 bedeuten.

10. Verfahren zur Herstellung von in einem der Ansprüche 1 bis 9 definierten Verbindungen der Formel IA bzw. IB, dadurch gekennzeichnet, dass man eine Verbindung der Formel II

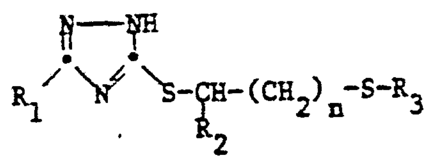

(II)

worin $R_1$, $R_2$, $R_3$ und n die entsprechenden, in einem der Ansprüche 1 bis 9 angegebenen Bedeutungen haben, in Gegenwart einer Base mit einem N,N-Dimethylcarbamoylhalid umsetzt.

11. Schädlingsbekämpfungsmittel enthaltend als aktive Komponente eine der in einem der Ansprüche 1 bis 9 definierten Verbindungen.

12. Verwendung von einer der in einem der Ansprüche 1 bis 9 definierten Verbindungen zur Bekämpfung von Schädlingen.

13. Verwendung nach Anspruch 12 zur Bekämpfung von pflanzenschädigenden Insekten.

14. Verbindungen der Formel II

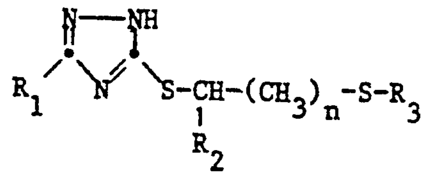

(II)

worin $R_1$ eine i-Propyl-, s-Butyl-, t-Butyl oder gegebenenfalls durch Methyl substituierte Cyclopropylgruppe, $R_2$ ein Wasserstoffatom oder eine Methylgruppe, $R_3$ eine $C_1$-$C_4$-Alkylgruppe und n die Zahl 0 oder 1 bedeuten.

15. Verbindungen nach Anspruch 1, worin $R_1$ eine i-Propyl-, s-Butyl-, t-Butyl- oder Cyclopropylgruppe bedeutet.

16. Verfahren zur Herstellung von in einem der Ansprüche 14 oder 15 definierten Verbindungen, dadurch gekennzeichnet, dass man eine Verbindung der Formel III

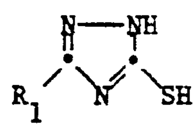

(III)

in Gegenwart einer Base mit einer Verbindung der Formel IV

$$Hal\text{-}CH(R_2)\text{-}(CH_2)_n\text{-}S\text{-}R_3 \qquad (IV)$$

umsetzt, worin $R_1$, $R_2$, $R_3$ und n die entsprechenden, in einem der Ansprüche 14 oder 15 definierten Bedeutungen haben und Hal ein Halogenatom darstellt.

0014734

Patentansprüche

(für Oesterreich)

1. Schädlingsbekämpfungsmittel enthaltend als aktive Komponente eine Verbindung der Formel IA bzw. IB

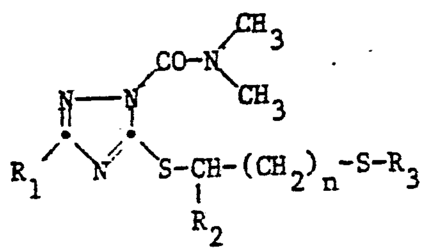

(IA)

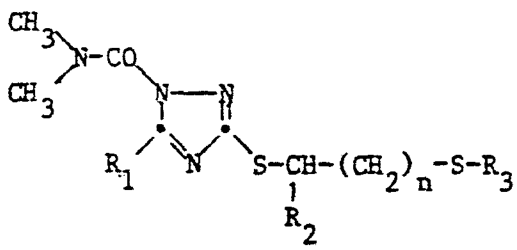

(IB)

worin $R_1$ eine i-Propyl-, s-Butyl-, t-Butyl- oder gegebenenfalls durch Methyl substituierte Cyclopropylgruppe, $R_2$ ein Wasserstoffatom oder eine Methylgruppe, $R_3$ eine $C_1$-$C_4$-Alkylgruppe und n die Zahl 0 oder 1 bedeuten.

2. Schädlingsbekämpfungsmittel nach Anspruch 1 enthaltend eine Verbindung der Formel IA bzw. IB, worin $R_1$ eine i-Propyl-, s-Butyl-, t-Butyl- oder Cyclopropylgruppe bedeutet.

3. Schädlingsbekämpfungsmittel nach Anspruch 1 enthaltend eine Verbindung der Formel IA bzw. IB, worin $R_1$ eine t-Butylgruppe bedeutet.

4. Schädlingsbekämpfungsmittel nach einem der Ansprüche 1 bis 3 enthaltend eine Verbindung der Formel IA bzw. IB, worin $R_2$ ein Wasserstoffatom und $R_3$ eine Methyl- oder Aethylgruppe bedeuten.

5. Schädlingsbekämpfungsmittel nach einem der Ansprüche 1 bis 4 enthaltend eine Verbindung der Formel IA bzw. IB, worin n die Zahl 0 bedeutet.

6. Schädlingsbekämpfungsmittel nach Anspruch 1 enthaltend eine Verbindung der Formel IA bzw. IB, worin $R_1$ eine t-Butylgruppe, $R_2$ ein Wasserstoffatom, $R_3$ eine Methylgruppe und n die Zahl 0 bedeuten.

7. Schädlingsbekämpfungsmittel nach Anspruch 1 enthaltend eine Verbindung der Formel IA bzw. IB, worin $R_1$ eine t-Butylgruppe, $R_2$ ein Wasserstoffatom, $R_3$ eine Methylgruppe und n die Zahl 1 bedeuten.

8. Schädlingsbekämpfungsmittel nach Anspruch 1 enthaltend eine Verbindung der Formel IA bzw. IB, worin $R_1$ eine 2-Methyl-cyclopropylgruppe, $R_2$ ein Wasserstoffatom, $R_3$ eine Methylgruppe und n die Zahl 0 bedeuten.

9. Schädlingsbekämpfungsmittel nach Anspruch 1 enthaltend eine Verbindung der Formel IA bzw. IB, worin $R_1$ eine 1-Methyl-cyclopropylgruppe, $R_2$ ein Wasserstoffatom, $R_3$ eine Methylgruppe und n die Zahl 1 bedeuten.

10. Verwendung von einer der in einem der Ansprüche 1 bis 9 definierten Verbindungen zur Bekämpfung von Schädlingen.

11. Verwendung nach Anspruch 10 zur Bekämpfung von pflanzenschädigenden Insekten.

12. Verfahren zur Herstellung von in einem der Ansprüche 1 bis 9 definierten Verbindungen der Formel IA und IB, dadurch gekennzeichnet, dass man eine Verbindung der Formel II

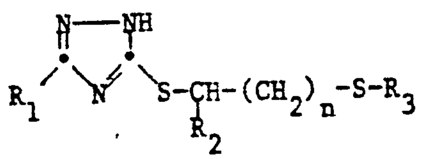

(II)

worin $R_1$, $R_2$, $R_3$ und n die entsprechenden, in einem der Ansprüche 1 bis 9 angegebenen Bedeutungen haben, in Gegenwart einer Base mit einem N,N-Dimethylcarbamylhalid umsetzt.

13. Verfahren zur Herstellung von im Anspruch 12 definierten Verbindungen der Formel II, dadurch gekennzeichnet, dass man eine Verbindung der Formel I

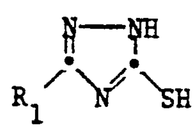

(III)

in Gegenwart einer Base mit einer Verbindung der Formel IV

$$Hal-CH(R_2)-(CH_2)_n-S-R_3 \qquad (IV)$$

umsetzt, worin $R_1$, $R_2$, $R_3$ und n die im Anspruch 12 definierten Bedeutungen haben und Hal ein Halogenatom darstellt.

Europäisches Patentamt

# EUROPÄISCHER RECHERCHENBERICHT

0014734

Nummer der Anmeldung

EP 79 10 3253

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | FR - A - 2 277 830 (BOOTS)<br>* Ansprüche *<br>-- | 1,10-13 | C 07 D 249/12<br>A 01 N 43/64 |
| | FR - A - 2 342 660 (GULF OIL)<br>* Ansprüche *<br>-- | 1,11-13 | |
| D | US - A - 3 308 131 (B.C. McKUSICK et al.)<br>* Spalten 1-3 *<br>---- | 1,10 | |

RECHERCHIERTE SACHGEBIETE (Int. Cl. 3)

C 07 D 249/12
A 01 N 43/64

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 28-01-1980 | CREMERS |